(19) Europäisches Patentamt
European Patent Office
Office européen des brevets

(11) Publication number: 0 248 573
A2

(12) # EUROPEAN PATENT APPLICATION

(21) Application number: 87304580.1

(22) Date of filing: 22.05.87

(51) Int. Cl.[3]: C 07 D 471/04
C 07 B 57/00
//(C07D471/04, 239:00, 221:00)

(30) Priority: 06.06.86 US 871539

(43) Date of publication of application:
09.12.87 Bulletin 87/50

(84) Designated Contracting States:
AT BE CH DE ES FR GB GR IT LI LU NL SE

(71) Applicant: THE TRUSTEES OF PRINCETON UNIVERSITY
Nassau Street
Princeton New Jersey 08544(US)

(72) Inventor: Taylor, Edward C.
288 Western Way
Princeton New Jersey 08544(US)

(72) Inventor: Shih, Chuan
8521 East Scarsdale Drive
Indianapolis Indiana 46256(US)

(72) Inventor: Beardsley, George Peter
Tonawanda Valley Box 192
Essex Connecticut 06426(US)

(72) Inventor: Fletcher, Stephen R.
11 Bowles Place Woughton-on-the-Green
Milton Keynes Buckinghamshire(GB)

(74) Representative: Jump, Timothy John Simon et al,
F.J. Cleveland and Company 40-43 Chancery Lane
London WC2A 1JQ(GB)

(54) **Diastereoisomeric tetrahydropyrido(2,3-d)pyrimidine derivatives.**

(57) The diastereoisomeric forms of N-(4-[2-(2-amino-4-hydroxy-5,6,7,8-tetrahydropyrido[2,3-d] -pyrimidin-6-yl)ethyl]-benzoyl)-L-glutamic acid are antineoplastic agents. The compounds are prepared by separation of the diastereoisomeric form of the correspondingly protected glutamic derivatives and hydrolytic or hydrogenolytic removal of carboxylic acid and/or amino protecting groups.



Croydon Printing Company Ltd.

0248573

# DIASTEREOISOMERIC TETRAHYDROPYRIDO[2,3-d]PYRIMIDINE DERIVATIVES

This invention pertains to two individual diastereoisomers of N-(4-[2-(5,6,7,8-tetrahydro-pyrido[2,3-d]pyrimidin-6-yl)alkyl]ethyl]benzoyl)-L-glutamic acid, each substantially free of all other diastereoisomers, to their preparation, and to their use as antineoplastic agent.

The folic acid antimetabolites aminopterin and amethopterin (also known as 10-methylaminopterin or methotrexate) are antineoplastic agents. These compounds inhibit enzymatic conversions involving metabolic derivatives of folic acid. Amethopterin, for example, inhibits dihydrofolate reductase, an enzyme necessary for the regeneration of tetrahydrofolate from the dihydrofolate which is formed during the conversion of 2-deoxyuridylate to thymidylate by the enzyme thymidylate synthetase.

Other derivatives of folic acid and aminopterin have been synthesized and tested as antimetabolites. Among these are compounds in which a methylene or methylidene group occupies a position in the molecule normally occupied by an imino or nitrilo group, respectively. These derivatives have varying degrees of antimetabolic activity. 10-Deazaaminopterin is highly active (Sirotak et al., Cancer Treat. Rep., 1978, 62, 1047) and 5-deazaaminopterin has activity similar to that of amethopterin (Taylor et al., J. Org. Chem., 1983, 48, 4852). 8,10-Dideazaaminopterin is reported to be active (U.S. Patent No. 4,460,591) and 5,8,10-trideazaaminopterin exhibits activity against mouse L1210 leukemia (Yan et al., J. Heterocycl. Chem.,

1979, 16, 541). 10-Deazafolic acid, on the other hand, shows no significant activity (Struck et al., J. Med. Chem., 1971, 14, 693) and 5-deazafolic acid is only weakly cytotoxic. 8,10-Dideazafolic acid is only marginally effective as a dihydrofolate reductase inhibitor (De Graw et al., "Chemistry and Biology of Pteridines", Elsevier, 1979, 229) and 5,8,10-trideazafolic acid also shows only marginal activity against mouse L1210 leukemia (Oatis et al., J. Med. Chem., 1977, 20, 1393). 5,10-Dideazaaminopterin and 5,10-dideaza-5,6,7,8-tetrahydroaminopterin, and the corresponding 5,10-dideazafolic acid derivatives are reported by Taylor et al., J. Med. Chem., 28:7, 914 (1985).

As described in South African Patent No. 86/1235 the compound N-(4-[2-(2-amino-4-hydroxy-5,6,7,8-tetrahydropyrido[2,3-d]pyrimidin-6-yl)ethyl]-benzoyl)-L-glutamic acid is a broad spectrum antineoplastic agent. Two chiral centers are present in this compound: the carbon atom in the 6-position of the tetrahydropyrido[2,3-d]pyrimidine ring and the alpha carbon atom in the glutamic acid group. Of the theoretical four forms of the compound, the use of an L-glutamic acid reagent in the initial coupling of the compounds as described in South African Patent No. 86/1235 reduces the possibilities to two. Both of these, however, are generated during the subsequent hydrogenation and consequently, upon removal of the protecting groups, the desired compound is produced as a mixture of the (S,S) and (R,S) diastereoisomers:

(S,S):

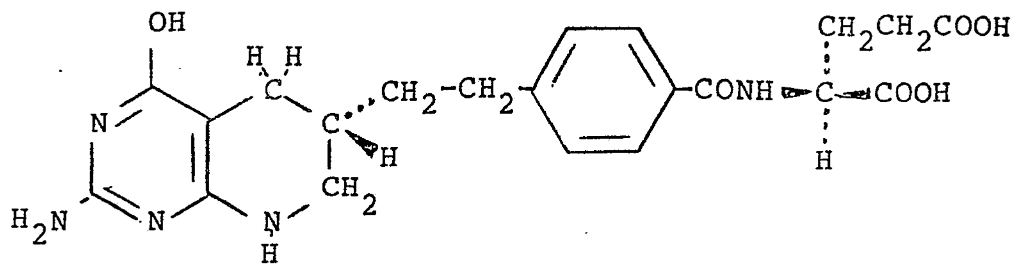

(R,S):

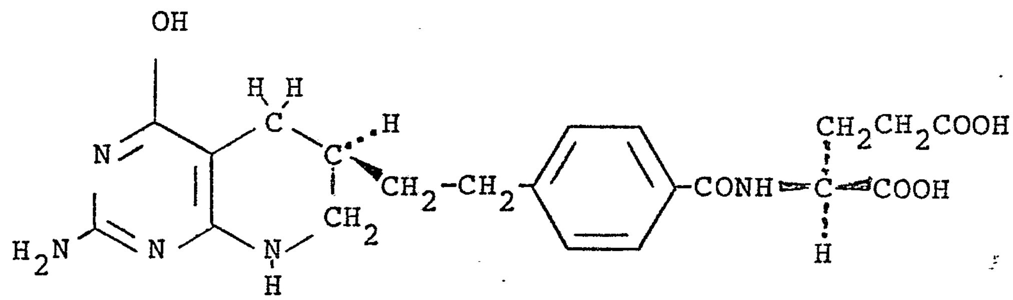

These diastereoisomers can be separated mechanically, as by chromatography, but such is impeded to some degree, particularly in large scale operations, by the low solubility of these compounds in most organic solvents. The mixture of diastereoisomers can be utilized therapeutically, both serving as substrates for relevant folate enzymes. Advantageously, however, since the compounds have somewhat distinct biological profiles, it is desirable to separate each in a form substantially free of the other; i.e., in a form having an optical purity of >95%.

A solution of a mixture of diastereoisomeric diethyl N-(4-[2-(2-acetamido-4-hydroxy-5,6,7,8-tetrahydropyrido[2,3-d]pyrimidin-6-yl)ethyl]benzoyl)-L-glutamate, or other protected derivatives, is treated with a chiral acid operable to form a salt therewith. The resultant diastereoisomeric salts are then separated

through one or more fractional crystallizations and thereafter the free base of the cationic moiety of at least one of the separated salts is liberated through treatment with a base and removal of the protecting groups. The liberation of the cation of the salt can be performed as a discrete step before or after the removal of the protecting groups, or concomitantly with the removal when such groups are susceptible to removal under basic conditions; i.e., basic hydrolysis.

Suitable chiral acids include the individual enantiomers of 10-camphorsulfonic acid, camphoric acid, alpha bromocamphoric acid, menthoxyacetic acid, tartaric acid, diacetyltartaric acid, malic acid, pyrrolidone-5-carboxylic acid, and the like.

There thus are obtained (i) the diastereoisomer of N-(4-[2(2-amino-4-hydroxy-5,6,7,8-tetrahydropyrido[2,3-d]pyrimidin- 6-yl)-ethyl]-benzoyl)-L-glutamic acid having a $[\alpha]^{25}_{589nm}$ of -21.06° which is substantially free of the diastereoisomer of N-(4-[2-(2-amino-4-hydroxy-5,6,7,8-tetrahydropyrido[2,3-d]-pyrimidin-6-yl)ethyl]-benzoyl)-L-glutamic acid having a $[\alpha]^{25}_{589nm}$ of +31.09° and (ii) the diastereoisomer of N-(4-[2-(2-amino-4-hydroxy-5,6,7,8-tetrahydropyrido-[2,3-d]pyrimidin-6-yl)-ethyl]-benzoyl)-L-glutamic acid having a $[\alpha]^{25}_{589nm}$ of +31.09° which in turn is substantially free of the diastereoisomer of N-(4-[2-(2-amino-4-hydroxy-5,6,7,8-tetrahydropyrido[2,3-d]pyrimidin-6-yl)ethyl]-benzoyl)L-glutamic acid having a $[\alpha]^{25}_{589nm}$ of -21.06°.

The removal of the protecting groups can be acheived through hydrolysis which is conducted at normal temperatures utilizing aqueous acid or base, such as for example, an aqueous alkali metal hydroxide, optionally in the presence of a water miscible organic solvent such as methanol, ethanol, tetrahydrofuran, dimethylformamide, and the like, or an acid, as for example trifluoroacetic acid. When base is used, the cationic

moiety of the salt is liberated and the product is formed as the dicationic glutamate salt which can be readily precipitated by adjustment of pH, as through acidification with, for example, acetic acid. The resulting products generally are high melting crystalline or microcrystalline solids.

Example 1

A mixture of 1.0 g of diethyl N-(4-[2-(2-acetamido-4-hydroxy-5,6,7,8-tetrahydropyrido[2,3-d]-pyrimidin-6-yl)ethyl]benzoyl)-L-glutamate and 880 mg of d(+)-10-camphorsulfonic acid in 50 mL of anhydrous ethanol is heated at reflux for 4 hours. The reaction mixture was allowed to cool to room temperature and to stand overnight. The white solid which formed was collected by filtration and fractionally crystallized six times in ethanol to yield 37 mg of diastereoisomer "B" of diethyl N-(4-[2-(2-acetamido-4-hydroxy-5,6,7,8-tetrahydropyrido[2,3-d]pyrimidin-6-yl)ethyl]benzoyl)-L-glutamate d(+)-10-camphorsulfonate, m.p. 223-225°C, $[\alpha]^{25}_{589nm}$ = -20.02°.

The solvent is removed from the mother liquor by evaporation and the solid which forms is recrystallized twice from ethanol to yield diastereoisomer "A" of diethyl N-(4-[2-(2-acetamido-4-hydroxy-5,6,7,8-tetrahydropyrido[2,3-d]pyrimidin-6-yl)ethyl]benzoyl)-L-glutamate d(+)-10-camphorsulfonate, $[\alpha]^{25}_{589nm}$ = +29.35°.

A solution of diastereoisomer "B" of diethyl N-(4-[2-(2-acetamido-4-hydroxy-5,6,7,8-tetrahydropyrido-[2,3-d]pyrimidin-6-yl)ethyl]benzoyl)-L-glutamate d(+)-10-camphorsulfonate in 50 mL of methanol containing 3 mL of 1N aqueous sodium hydroxide was stirred at room temperature for 72 hours. Addition of 2 mL of acetic acid followed by centrifugation yielded diastereoisomer "B" of N-(4-[2-(2-amino-4-hydroxy-5,6,7,8-tetrahydropyrido[2,3-d]pyrimidin-6-yl)ethyl]benzoyl)-L-glutamic

acid, mp 224-227°C (dec.) $[\alpha]^{25}_{589nm}$ = -21.0581° (c = 0.636, 0.1N NaOH); $\lambda_{max}$ 278nm, 222nm; NMR ($CDCl_3$) delta 1.85 (m, 2H), 1.98 (m, 1H), 2.25 (m, 1H), 2.45 (m, 1H), 2.68 (m 1H), 2.92 (m, 5H), 3.25 (t, J = 10Hz, 1H), 3.82 (d, J = 10Hz, 1H), 513 (m, 1H) 7.43 (d, J = 9 Hz, 2H), 7.84 (d, J = 9 Hz, 2H). The optical purity is >97%.

By following the above procedure, but utilizing diastereoisomer "A" of diethyl N-(4-[2-(2-acetamido-4-hydroxy-5,6,7,8-tetrahydropyrido[2,3-d]-pyrimidin-6-yl)ethyl]benzoyl)-L-glutamate d(+)-10-camphorsulfonate in place of diastereoisomer "B", there is obtained diastereoisomer "A" of N-(4-[2-(2-amino-4-hydroxy-5,6,7,8-tetrahydropyrido[2,3-d]pyrimidin-6-yl)ethyl]benzoyl)-L-glutamic acid, mp 253-255°C (dec.) $[\alpha]^{25}_{589nm}$ = +31.0915° (c = 3.605, 0.1N NaOH); $\lambda_{max}$ 278nm, 222nm; NMR ($CDCl_3$) delta 1.85 (m, 2H), 1.98 (m, 1H), 2.25 (m, 1H), 2.45 (m, 1H), 2.68 (m 1H), 2.92 (m, 5H), 3.25 (t, J = 10Hz, 1H), 3.82 (d, J = 10Hz, 1H), 513 (m, 1H) 7.43 (d, J = 9 Hz, 2H), 7.84 (d, J = 9 Hz, 2H). The optical purity is >97%.

## Example 2

The $IC_{50}$ value was determined in whole cell human leukemia cell lines, CCRF-CEM, for diastereoisomer "A" and diastereoisomer "B". Results of these experiments are as follows:

| Compound | $IC_{50}$ (mcg/mL) |
|---|---|
| Diastereoisomer "A" | $2.6 \times 10^{-3}$ |
| Diastereoisomer "B" | $3.4 \times 10^{-3}$ |

## Example 3

B-16 melanoma tumor cells were implanted subcutaneously in the axillary region of C57BL/G mice. Groups of ten mice were used for each dosage. Following

daily intraperitoneal administration of each of N-(4-[2-(2-amino-4-hydroxy-5,6,7,8-tetrahydropyrido-[2,3-d]pyrimidin-6yl)ethyl]benzoyl)-L-glutamic acid, diastereoisomer "A", and diastereoisomer "B", the mass of the control tumor (receiving only diluent) was measured after ten days and compared to those of animals receiving the test compound to calculate percentage of inhibition. The results are as follows:

| Compound | Dose (mg/kg) | % Inhibition | Survivors |
|---|---|---|---|
| Mixture of Isomers A and B | 1.60 | 5 | 9/9 |
| | 3.12 | 12 | 9/9 |
| | 6.25 | 13 | 9/9 |
| | 12.50 | 50 | 9/9 |
| Isomer A | 1.60 | 47 | 9/9 |
| | 3.12 | 24 | 9/9 |
| | 6.25 | 0 | 9/9 |
| | 12.50 | 0 | 9/9 |
| Isomer B | 1.60 | 71 | 10/10 |
| | 3.12 | 91 | 10/10 |
| | 6.25 | 98 | 10/10 |
| | 12.50 | 100 | 10/10 |
| | 25.00 | 100 | 10/10 |
| | 50.00 | 100 | 9/10 |
| | 100.00 | 100 | 8/10 |
| | 200.00 | 100 | 6/10 |

Example 4

Groups of 10 C3H female mice were innoculated intraperitoneally in the axillary region with 6C3HED lymphosarcoma cells. The test compound is then administered in Emulphor IP (0.5 mL) for 8 days. Controls received identical treatment without test compound.

| Compound | Dose (mg/kg) | % Inhibition | Survivors |
|---|---|---|---|
| Isomer A | 6.00 | 74 | 10/10 |
| | 12.50 | 89 | 10/10 |
| | 25.00 | 100 | 10/10 |
| | 50.00 | 100 | 10/10 |
| | 100.00 | 100 | 10/10 |
| Isomer B | 3.60 | 71 | 10/10 |
| | 6.25 | 84 | 10/10 |
| | 12.50 | 89 | 10/10 |
| | 25.00 | 99 | 10/10 |
| | 50.00 | 100 | 10/10 |
| | 100.00 | 100 | 8/10 |

Example 5

C3H mammary adenocarcinoma cells were implanted in groups of C3H mice and Isomer B then was administered as indicated with the control animals receiving only the diluent.

Continuous infusion (2 mL/day) in 1% $NaHCO_3$ Daily for 5 days.

| Dose (mg/kg) | % Inhibition | Survivors |
|---|---|---|
| 0.62 | 20 | 5/5 |
| 1.25 | 59 | 6/6 |
| 2.50 | 93 | 6/6 |
| 5.00 | Toxic | 0/6 |

Intraperitoneally (0.5 mL/day) In Emulphor Daily for 10 days

| Dose (mg/kg) | % Inhibition | Survivors |
|---|---|---|
| 3.60 | 76 | 10/10 |
| 6.25 | 93 | 10/10 |
| 12.50 | 99 | 8/10 |
| 25.00 | 100 | 7/10 |
| 50.00 | 100 | 8/10 |
| 100.00 | Toxic | 0/10 |

Intraperitoneally (0.5 mL/day)
In Emulphor Days 1,3,5,7 & 9 only

| Dose (mg/kg) | % Inhibition | Survivors |
|---|---|---|
| 50 | 91 | 10/10 |
| 100 | 96 | 10/10 |
| 200 | 100 | 10/10 |
| 400 | 100 | 8/10 |

Intraperitoneally (0.6 mL/day)
In Emulphor Days 1,3,5,7 & 9 only

| Dose (mg/kg) | % Inhibition | Survivors |
|---|---|---|
| 6.25 | 39 | 10/10 |
| 12.50 | 21 | 10/10 |
| 25.00 | 59 | 10/10 |
| 50.00 | 60 | 10/10 |
| 100.00 | 94 | 10/10 |

## Example 6

X5563 plasma cell myeloma cells were implanted in groups of C3H mice and Isomer B then was administered as indicated with the control animals receiving only the diluent.

Intraperitoneally (0.6 mL/day)
In Emulphor Daily for 10 days

| Dose (mg/kg) | % Inhibition | Survivors |
|---|---|---|
| 3.60 | 62 | 10/10 |
| 6.25 | 76 | 7/10 |
| 12.50 | 90 | 10/10 |
| 25.00 | 99 | 8/10 |
| 50.00 | 99 | 7/9 |
| 100.00 | Toxic | 0/10 |

CLAIMS:

1. The process which comprises treating a solution of a mixture of diastereoisomeric compounds of the formula:

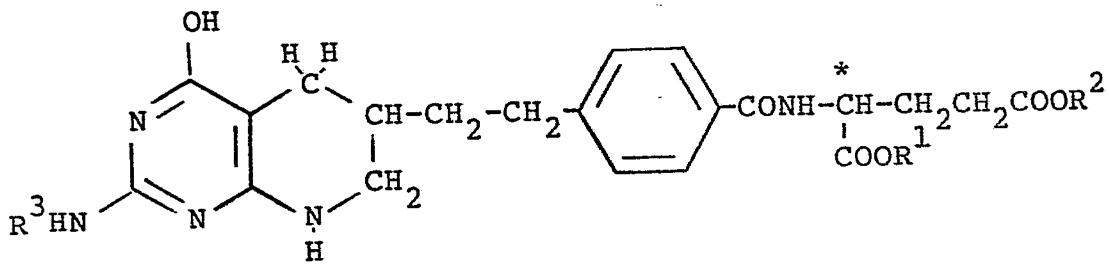

wherein

$R^1$ and $R^2$ are the same or different carboxylic acid protecting group;

$R^3$ is hydrogen or an amino protecting group; and the configuration about the carbon atom designated * is L;

with a chiral acid operable to form a salt with said mixture, separating the resultant two diastereoisomeric salts of said acid and said compound, and thereafter liberating as the free base the cationic moiety of at least one of said separated salts through treatment with a base and removing said protecting groups, said liberation being performed either as a discrete step before or after said removal of the protecting groups, or concomitantly with said removal, to yield at least one of

(i) the diastereoisomer of N-(4-[2-(2-amino-4-hydroxy-5,6,7,8-tetrahydropyrido[2,3-d]pyrimidin-6-yl)-ethyl]-benzoyl)-L-glutamic acid which has a $[\alpha]^{25}_{589nm}$ of -21.06° substantially free of the diastereoisomer of N-(4-[2-(2-amino-4-hydroxy-5,6,7,8-tetrahydropyrido-[2,3-d]pyrimidin-6-yl)ethyl]benzoyl)-L-glutamic acid which has a $[\alpha]^{25}_{589nm}$ of +31.09° and

(ii) the diastereoisomer of N-(4-[2-(2-amino-4-hydroxy-5,6,7,8-tetrahydropyrido[2,3-d]pyrimidin-6-yl)-ethyl]-benzoyl)-L-glutamic acid which has a $[\alpha]^{25}_{589nm}$ of +31.09° substantially free of the diastereoisomer of N-(4-[2-(2-amino-4-hydroxy-5,6,7,8-tetrahydropyrido[2,3-d]pyrimidin-6-yl)ethyl]-benzoyl)-L-glutamic acid which has a $[\alpha]^{25}_{589nm}$ of -21.06°.

2. A compound selected from the group consisting of:

(i) the diastereoisomer of N-(4-[2-(2-amino-4-hydroxy-5,6,7,8-tetrahydropyrido[2,3-d]-pyrimidin-6-yl)ethyl]-benzoyl)-L-glutamic acid having a $[\alpha]^{25}_{589nm}$ of -21.06° and being substantially free of the diastereoisomer of N-(4-[2-(2-amino-4-hydroxy-5,6,7,8-tetrahydropyrido[2,3-d]pyrimidin-6-yl)ethyl]-benzoyl)-L-glutamic acid which has a $[\alpha]^{25}_{589nm}$ of +31.09°;

(ii) the 4(3H)-oxo tautomeric form thereof; and

(iii) a pharmaceutically acceptable alkali metal, alkaline earth, non-toxic metal, ammonium, or substituted ammonium salt thereof which is substantially free of the corresponding salt of that diastereoisomer which has a $[\alpha]^{25}_{589nm}$ of +31.09°.

3. The diastereoisomer of N-(4-[2-(2-amino-4-hydroxy-5,6,7,8-tetrahydropyrido[2,3-d]pyrimidin-6-yl)-ethyl]-benzoyl)-L-glutamic acid having a $[\alpha]^{25}_{589nm}$ of -21.06° and being substantially free of the diastereoisomer of N-(4-[2-(2-amino-4-hydroxy-5,6,7,8-tetrahydropyrido[2,3-d]pyrimidin-6-yl)ethyl]benzoyl)-L-glutamic acid which has a $[\alpha]^{25}_{589nm}$ of +31.09°.

4. A compound selected from the group consisting of:

(i) the diastereoisomer of N-(4-[2-(2-amino-4-hydroxy-5,6,7,8-tetrahydropyrido[2,3-d]-pyrimidin-6-yl)ethyl]-benzoyl)-L-glutamic acid having a $[\alpha]^{25}_{589nm}$ of +31.09° and being substantially free of the diastereoisomer of N-(4-[2-(2-amino-4-hydroxy-5,6,7,8-tetrahydropyrido[2,3-d]pyrimidin-6-yl)ethyl]-benzoyl)-L-glutamic acid which has a $[\alpha]^{25}_{589nm}$ of -21.06°;

(ii) the 4(3H)-oxo tautomeric form thereof;

and

(iii) a pharmaceutically acceptable alkali metal, alkaline earth, non-toxic metal, ammonium, or substituted ammonium salt thereof which is substantially free of the corresponding salt of that diastereoisomer which has a $[\alpha]^{25}_{589nm}$ of -21.06°.

5. The diastereoisomer of N-(4-[2-(2-amino-4-hydroxy-5,6,7,8-tetrahydropyrido[2,3-d]pyrimidin-6-yl)-ethyl]-benzoyl)-L-glutamic acid having a $[\alpha]^{25}_{589nm}$ of +31.09° and being substantially free of the diastereoisomer of N-(4-[2-(2-amino-4-hydroxy-5,6,7,8-tetrahydropyrido[2,3-d]pyrimidin-6-yl)ethyl]benzoyl)-L-glutamic acid which has a $[\alpha]^{25}_{589nm}$ of -21.06°.